# EUROPEAN PATENT APPLICATION

(11) **EP 4 790 420 A1**
(43) Date of publication of application: **12.08.2026**
(21) Application number: 24874739.6
(22) Date of filing: 04.10.2024
(51) Int. Cl.: G01N 33/68, G01N 33/15, G01N 33/50

(54) **METHOD FOR EVALUATING LONG-TERM HIGH ONSET RISK OF CEREBROVASCULAR, CARDIOVASCULAR, AND RENAL EVENTS USING SOLUBLE CLEC-2**

(30) Priority: 06.10.2023 JP 2023174721
(71) Applicant: PHC Corporation, Toon-shi Ehime 791-0395 (JP)
(72) Inventor: IZAWA, Hideo, Toyoake-shi, Aichi 470-1192 (JP); TSUBOI, Naotake, Toyoake-shi, Aichi 470-1192 (JP); HASEGAWA, Midori, Toyoake-shi, Aichi 470-1192 (JP); ISHII, Junichi, Toyoake-shi, Aichi 470-1192 (JP); NARUSE, Hiroyuki, Toyoake-shi, Aichi 470-1192 (JP); KAWAI, Hideki, Toyoake-shi, Aichi 470-1192 (JP); KITAGAWA, Fumihiko, Toyoake-shi, Aichi 470-1192 (JP); KAWAMURA, Masahide, Toon-shi, Ehime 791-0395 (JP)
(74) Representative: Cohausz & Florack
(86) International application number: PCT/JP2024/035597
(87) International publication number: WO 2025/075139

(57) **Abstract**

Provided is a technique capable of accurately and conveniently predicting the long-term onset risk of cerebrovascular, cardiovascular, and renal events. Furthermore, by use of the technique, provided is a technique for selecting a treatment method for increasing preventative benefits for subject patients. The techniques evaluate the long-term high onset risk of cerebrovascular, cardiovascular, and renal events based on the concentration of soluble CLEC-2 in the subject's blood.

## Description

### TECHNICAL FIELD

The present invention relates to a method for evaluating long-term high onset risk of cerebrovascular, cardiovascular, and renal events using soluble CLEC-2 (sCLEC-2).

### BACKGROUND ART

Cerebrovascular, cardiovascular, and renal events refer to the onset of cerebrovascular or cardiovascular diseases, such as stroke, ischemic heart diseases (myocardial infarction or the like), and arteriosclerosis obliterans, due to arteriosclerosis or thrombosis; the initiation of hemodialysis; or the like. Cerebrovascular or cardiovascular diseases are a critically important group of diseases: these diseases are major causes of death like cancer in Japan, and the leading cause of death globally. Accurately evaluating this risk of onset and implementing appropriate prevention and treatment is extremely important.

As risk factors for cerebrovascular, cardiovascular, and renal events, for example, diabetes, chronic kidney disease (CKD), hypertension, dyslipidemia, obesity, and smoking are known. Managing each of these factors is crucial for reducing risk. Furthermore, the risk increases when multiple risk factors are present simultaneously. Markers exist to evaluate these risk factors, and corresponding preventive and therapeutic measures are being implemented.

For example, dyslipidemia has risk markers such as LDL cholesterol, and if these show abnormalities, there are drugs like statins to improve dyslipidemia. For diabetes, there are risk markers such as blood glucose levels and hemoglobin A1C, and if these are abnormal, measures such as antidiabetic drugs, diet, and exercise are implemented. Furthermore, for hypertension, treatments such as antihypertensive drugs, and for smokers, smoking cessation, are implemented as preventive measures against cerebrovascular, cardiovascular, and renal events, tailored to each individual risk.

However, even with such individualized therapeutic and preventive measures in place, patients with diabetes, hypertension, chronic kidney disease, or other conditions remain at risk for cerebrovascular, cardiovascular, and renal events. Therefore, evaluating and managing the risk of cerebrovascular, cardiovascular, and renal events remains critically important.

It is well known that the mechanism underlying major cerebrovascular and cardiovascular events such as cerebral infarction, myocardial infarction, and arteriosclerosis obliterans is vascular occlusion caused by thrombus formation. Antithrombotic therapy is well established as a preventive measure against these events. Antiplatelet therapy is used when platelets are considered the primary cause of thrombosis, such as in arterial thrombosis, while anticoagulants are used when the coagulation system is considered the primary cause of thrombosis, such as in venous thrombosis.

In the present specification, the risk group and high-risk group for cerebrovascular, cardiovascular, and renal events are defined as follows.

The risk group is defined as those who are generally not treated with antithrombotic therapy, despite being considered at higher risk than healthy individuals for cerebrovascular, cardiovascular, and renal events. This risk group includes patients with, for example, diabetes, chronic kidney disease, hypertension, dyslipidemia, obesity, and smoking habits.

The high-risk group is defined as those with a higher risk of cerebrovascular, cardiovascular, and renal events than the risk group, for whom antithrombotic therapy is recommended. This high-risk group includes, for example, patients with a history of cerebral infarction, a history of myocardial infarction, a history of arteriosclerosis obliterans, angina pectoris, and atrial fibrillation, and those after artificial valve replacement.

Antithrombotic therapy suppresses thrombus formation to prevent thrombosis, but as a side effect, it may induce bleeding disorders due to the suppression of thrombus formation. Therefore, it is important to consider the balance between the benefits of prevention and the risks of side effects in antithrombotic therapy. Therefore, administration of antiplatelet drugs is used, for high-risk group diseases particularly prone to causing myocardial infarction, such as angina pectoris, or for the treatment or secondary prevention of high-risk patients such as those with a history of cerebral infarction, myocardial infarction, or arteriosclerosis obliterans. On the other hand, it is not generally used for primary prevention (prevention in individuals without a history of cerebrovascular or cardiovascular diseases) in risk patients such as those with diabetes, hypertension, or chronic kidney disease (CKD). Similarly, anticoagulant therapy is currently used, as thrombosis prevention, only for high-risk patients such as those with atrial fibrillation or those after artificial valve replacement. This point is also described in the Japanese version of the CKD clinical practice guidelines (Non-patent literature 1): "It is unclear whether aspirin (an antiplatelet agent) administration is beneficial for suppressing cerebrovascular or cardiovascular events in patients with CKD stages G3b to 5. On the other hand, the possibility that aspirin administration increases the risk of bleeding complications cannot be denied." As such, administering aspirin requires a more accurate risk evaluation.

Since antiplatelet agents are known to be effective for arteriosclerotic thrombosis, it can be inferred that monitoring changes in platelets would be a suitable risk prediction marker. For this purpose, it has been proposed to measure markers that observe platelet activation or markers that measure platelet aggregation ability. However, markers that measure platelet activity are difficult to measure operationally, and none are widely used clinically. There are no markers that predict cerebrovascular, cardiovascular, and renal events. Platelet aggregation test was also expected as a risk prediction marker, but its predictive capability was low and it did not reach practical application. The technology described in Patent literature 1, which measures the rate of decrease in platelet count, has also not reached practical application.

Patent literature 2 discloses that the concentration of soluble CLEC-2 (sCLEC-2), a platelet activation marker, represents platelet activation, and that its measurement values increase in thrombosis. Furthermore, if platelet-related marker values change, it is considered that thrombosis has already begun, and sCLEC-2 concentration is thought to indicate an increased imminent risk, that is, a heightened short-term risk (Patent literature 3). Furthermore, the technology disclosed in Patent literature 2 has not yet reached practical application. That is, a platelet-based thrombosis risk marker technology for short-term cerebrovascular, cardiovascular, and renal events has not yet been established.

Activation of the coagulation system is also important as a cause of thrombosis. Thrombus formation progresses through the interaction between platelets and the coagulation system. While it is said that the arterial thrombosis is primarily driven by platelets and the venous thrombosis by the coagulation system (fibrin formation), any thrombus contains both platelets and fibrin. Therefore, coagulation system markers may also serve as risk markers for the onset of cerebrovascular, cardiovascular, and renal events. A representative example is D-Dimer. While D-Dimer can serve as a marker for the onset of cerebrovascular, cardiovascular, and renal events such as cardiogenic cerebral infarction or aortic dissection, it is not generally used as a risk marker for cerebrovascular, cardiovascular, and renal events. For example, in Non-patent literature 2, D-Dimer is used to evaluate cardiovascular event risk. The D-Dimer tertiles for the patient group with stable coronary artery disease (a high-risk disease for cardiovascular events) are (<0.32, 0.32-0.61, >0.62 µg/mL). Patients in the third tertile (>0.62 µg/mL) have a higher onset risk of cardiovascular events compared to patients in the first tertile (<0.32 µg/mL). The upper limit of normal for a D-Dimer measurement reagent used in this study is 1.0 µg/mL. Therefore, since the risk evaluation threshold falls within the normal range of D-Dimer values, many healthy individuals would be classified into the high-risk group. Consequently, it is not suitable as a risk evaluation marker. Thus, D-Dimer is not considered an accurate marker for the onset risk of cerebrovascular, cardiovascular, and renal events.

As described above, there is currently no established technology capable of evaluating the onset risk of cerebrovascular, cardiovascular, and renal events from the perspective of thrombosis. Furthermore, there is no suggestion of a thrombosis risk marker capable of evaluating the long-term onset risk of cerebrovascular, cardiovascular, and renal events from the perspective of thrombosis.

### CITATION LIST

### PATENT LITERATURE

[Patent literature 1] JP 2022-73652 A
[Patent literature 2] JP 6078845 B
[Patent literature 3] WO 2022/255348
[Patent literature 4] WO 2021/172493

### NON-PATENT LITERATURE

[Non-patent literature 1] Clinical Practice Guidelines for the Prevention of Progression of Chronic Kidney Disease (Stage G3b-G5) and for Smooth Transition to Renal Replacement Therapy, 2015, Japanese Society of Nephrology
[Non-patent literature 2] H. Naruse et al. Circulation Journal 2017 81: 1506-1513

### SUMMARY OF INVENTION

### TECHNICAL PROBLEM

An object is to provide a technique capable of accurately and conveniently predicting the long-term onset risk of cerebrovascular, cardiovascular, and renal events, for which no useful means have been found despite the expectation of a large number of potential patients. In particular, among patients with diseases such as diabetes, hypertension, and chronic kidney disease, which are considered risk factors for cerebrovascular, cardiovascular, and renal events, it is to provide a technique that predicts patients with the long-term high onset risk of such events. Furthermore, by use of these techniques, it is to provide a technique for selecting a treatment method for enhancing the benefits for subject patients in prevention.

### SOLUTION TO PROBLEM

The present inventors conducted intensive studies to solve the problems, and as a result, among high-risk groups for cerebrovascular, cardiovascular, and renal events, the group with elevated blood sCLEC-2 concentrations was found to have a particularly high long-term risk of developing cerebrovascular, cardiovascular, and renal events. In addition to this finding, by using the combination of D-Dimer, a coagulation marker, with sCLEC-2; furthermore, the combination of sCLEC-2, D-Dimer, and platelet count; and particularly, an index value such as (blood sCLEC-2 concentration) × (blood D-Dimer concentration), or (blood D-Dimer concentration) × (blood sCLEC-2 concentration) / (platelet count); the present inventors found that patient groups with a particularly high long-term onset risk of cerebrovascular, cardiovascular, and renal events could be extracted, and thus completed the present invention.

Since most cerebrovascular, cardiovascular, and renal events are primarily caused by vascular occlusion due to thrombus formation, it is well known that biomarkers associated with fibrin (a product of the coagulation system) or platelets, which are the main components of thrombus, often show abnormal values at the time of onset of cerebrovascular, cardiovascular, and renal events. This is natural, because thrombi are often already present at the time of onset. As such markers, in the fibrin (coagulation) system, for example, D-Dimer, FDP, soluble fibrin, and thrombin-antithrombin complex are known, and in the platelet system, for example, sCLEC-2, β-thromboglobulin, platelet factor 4, soluble p-selectin, and platelet aggregation test are known. However, to be useful as long-term predictive markers for cerebrovascular, cardiovascular, and renal events, they must show abnormal values outside the normal ranges well before the onset of these events. There is no evidence that these existing markers can serve as long-term predictors of cerebrovascular, cardiovascular, and renal events, and consequently, they are not used for this purpose. The ability to evaluate the long-term high onset risk for cerebrovascular, cardiovascular, and renal events based on sCLEC-2 concentration, the combination of sCLEC-2 concentration and platelet count, the combination of sCLEC-2 concentration and D-Dimer concentration, and the combination of sCLEC-2 concentration, D-Dimer concentration, and platelet count is a surprising finding that could not have been predicted from previous knowledge. Furthermore, it becomes possible to readily recommend treatment methods to enhance the benefits of prevention for target patients, making the effects and significance of the present invention exceptionally high.

In the presence of thrombi or during thrombus formation, the aforementioned markers show abnormal values. However, the present inventors found that when new indicators, sCLEC-2 concentration alone, or the combination of sCLEC-2 concentration with platelet count, the combination of sCLEC-2 concentration with D-Dimer concentration, or the combination of sCLEC-2 concentration, D-Dimer concentration, and platelet count, showed abnormal values, the incidence rate of cerebrovascular, cardiovascular, and renal events up to 24 months after the measurement point was 3 to 8 times higher compared to cases without such abnormalities. Since thrombi are generally thought to form immediately before onset, thrombus-related markers that can predict onset 24 months in advance have not existed until now. In fact, existing platelet activation markers or platelet aggregation capacity are not known to serve as risk markers for cerebrovascular, cardiovascular, and renal events when used alone.

For example, if platelet-related markers such as sCLEC-2 or platelet count are abnormal, it is generally considered that thrombosis is occurring, thereby increasing short-term risk (Patent literature 3). On the other hand, as described in the Examples below, the present inventors found that the sCLEC-2 high-value group exhibited an increased cumulative incidence rate of cerebrovascular, cardiovascular, and renal events at a nearly constant rate over 24 months after measurement. If elevated sCLEC-2 levels indicate thrombus formation causing vascular occlusion occurring at the time of blood collection or shortly thereafter, it is predicted that the frequency of cerebrovascular, cardiovascular, and renal events will increase within a relatively short period. However, the Kaplan-Meier curves showed no phenomenon of a high incidence rate in incidence rates, and the cumulative incidence rate increased almost linearly over the 24-month period. Therefore, the present inventors have discovered that sCLEC-2 and its related indicators in the present invention reflect the long-term risk of subjects.

One example of a threshold (cut-off value) for judging long-term high onset risk of cerebrovascular, cardiovascular, and renal events based on sCLEC-2 concentration was 250 pg/mL. Since the sCLEC-2 concentration in the blood of healthy individuals is 200 pg/mL or less, very few healthy individuals would be classified as high risk at this threshold. It was also found that setting an appropriate sCLEC-2 cutoff value at 200 pg/mL or more would significantly reduce false positives, where low-risk individuals such as healthy individuals are incorrectly classified as high risk.

Furthermore, as described in the Examples below, to evaluate long-term high onset risk for cerebrovascular, cardiovascular, and renal events, it is preferable to evaluate thrombosis risk markers by evaluating the platelet system, or by simultaneously evaluating both the platelet system and the coagulation system. sCLEC-2 concentration can evaluate platelet status and may serve as a long-term onset risk marker for cerebrovascular, cardiovascular, and renal events on its own. However, combining sCLEC-2 concentration with D-Dimer concentration has been found to yield an even more useful long-term onset risk marker for cerebrovascular, cardiovascular, and renal events. Furthermore, combining sCLEC-2 concentration with platelet count enables accurate evaluation of platelet activity (Patent literature 4). Additionally, combining sCLEC-2 concentration with D-Dimer concentration and/or platelet count yields index values such as (blood sCLEC-2 concentration) × (blood D-Dimer concentration), (blood sCLEC-2 concentration) / (platelet count), or (blood D-Dimer concentration) × (blood sCLEC-2) / (platelet count) can serve as long-term high onset risk markers for cerebrovascular, cardiovascular, and renal events.

In summary, the present inventors have found that blood sCLEC-2 concentration alone, the combination of blood sCLEC-2 concentration with blood D-Dimer concentration, the combination of blood sCLEC-2 concentration with platelet count, the combination of blood sCLEC-2 concentration, blood D-Dimer concentration, and platelet count, in particular, index values such as (blood sCLEC-2 concentration) × (blood D-Dimer concentration), (blood sCLEC-2 concentration) / (platelet count), or (blood D-Dimer concentration) × (blood sCLEC-2 concentration) / (platelet count) can extract the long-term high onset risk group for cerebrovascular, cardiovascular, and renal events. The present invention is based on these findings.

The present invention provides the followings:
[1] A method for evaluating a long-term high onset risk of cerebrovascular, cardiovascular, and renal events based on a soluble CLEC-2 concentration in a subject's blood.
[2] The method of [1], for evaluating a long-term high onset risk of cerebrovascular, cardiovascular, and renal events based on a soluble CLEC-2 concentration in a subject's blood, said method comprising:
   (1) providing a blood sample derived from a subject;
   (2) determining a soluble CLEC-2 concentration in the blood sample; and
   (3) correlating the soluble CLEC-2 concentration with a long-term risk of onset for cerebrovascular, cardiovascular, and renal events in the subject.
[3] The method of [1] or [2], wherein the subject is a patient suspected of having a disease associated with a risk of cerebrovascular, cardiovascular, and renal events or a patient diagnosed with the disease.
[4] The method of any one of [1] to [3], wherein the disease associated with a risk of cerebrovascular, cardiovascular, and renal events is selected from the group consisting of chronic kidney disease, diabetes, hypertension, dyslipidemia, chronic maintenance dialysis, and atherosclerotic diseases.
[5] The method of any one of [1] to [4], wherein the long-term high onset risk of cerebrovascular, cardiovascular, and renal events is evaluated based on, in addition to the soluble CLEC-2 concentration in the subject's blood, a D-Dimer concentration in the subject's blood.
[6] The method of [5], wherein the long-term high onset risk of cerebrovascular, cardiovascular, and renal events is evaluated based on a value calculated by multiplying the soluble CLEC-2 concentration by the D-Dimer concentration in the subject's blood.
[7] The method of any one of [1] to [4], wherein the long-term high onset risk of cerebrovascular, cardiovascular, and renal events is evaluated based on, in addition to the soluble CLEC-2 concentration in the subject's blood, a subject's platelet count.
[8] The method of [7], wherein the long-term high onset risk of cerebrovascular, cardiovascular, and renal events is evaluated based on a value calculated by dividing the soluble CLEC-2 concentration in the subject's blood by the platelet count.
[9] The method of [7], wherein the long-term high onset risk of cerebrovascular, cardiovascular, and renal events is evaluated based on, in addition to the soluble CLEC-2 concentration in the subject's blood and the platelet count, a D-Dimer concentration in the subject's blood.
[10] The method of [9], wherein the long-term high onset risk of cerebrovascular, cardiovascular, and renal events is evaluated based on a value represented by the following formula (1): $\left\{\left(soluble CLEC-2\right)\times \left(D-Dimer concentration\right)\right\} / \left(platelet count\right)$
[11] A method for evaluating a long-term high onset risk of cerebrovascular, cardiovascular, and renal events by the method of any one of [1] to [10], and for selecting an antithrombotic drug.

Furthermore, the present invention includes:
- a method for evaluating a long-term high onset risk of cerebrovascular, cardiovascular, and renal events based on a soluble CLEC-2 concentration (i.e., for example, soluble CLEC-2 concentration alone, or combinations of soluble CLEC-2 concentration with D-Dimer concentration and/or platelet count) in a subject's blood;
- a method for evaluating a long-term high onset risk of cerebrovascular, cardiovascular, and renal events based on a soluble CLEC-2 concentration in a subject's blood, said method comprising:
   (1) providing a blood sample derived from a subject;
   (2) determining a soluble CLEC-2 concentration in the blood sample; and
   (3) correlating the soluble CLEC-2 concentration with a long-term risk of onset for cerebrovascular, cardiovascular, and renal events in the subject;
- a method for assisting in an evaluation of a long-term high onset risk of cerebrovascular, cardiovascular, and renal events based on a soluble CLEC-2 concentration (i.e., for example, soluble CLEC-2 concentration alone, or combinations of soluble CLEC-2 concentration with D-Dimer concentration and/or platelet count) in a subject's blood;
- a method for measuring (or determining) a soluble CLEC-2 concentration (i.e., for example, soluble CLEC-2 concentration alone, or combinations of soluble CLEC-2 concentration with D-Dimer concentration and/or platelet count) in a subject's blood, for evaluating a long-term high onset risk of cerebrovascular, cardiovascular, and renal events;
- an in vitro method for evaluating a long-term high onset risk of cerebrovascular, cardiovascular, and renal events, characterized by measuring (or determining) a soluble CLEC-2 concentration (i.e., for example, soluble CLEC-2 concentration alone, or combinations of soluble CLEC-2 concentration with D-Dimer concentration and/or platelet count) in a subject's blood;
- a use of an antibody capable of measuring (or determining) a soluble CLEC-2 concentration in the manufacture of a kit for evaluating a long-term high onset risk of cerebrovascular, cardiovascular, and renal events;
- a use of an antibody capable of measuring (or determining) a soluble CLEC-2 concentration and an antibody capable of measuring (or determining) a D-Dimer concentration in the manufacture of a kit for evaluating a long-term high onset risk of cerebrovascular, cardiovascular, and renal events; and
- a method for measuring (or determining) a soluble CLEC-2 concentration (i.e., for example, soluble CLEC-2 concentration alone, or combinations of soluble CLEC-2 concentration with D-Dimer concentration and/or platelet count) in a subject's blood, to provide information necessary for an evaluation of a long-term high onset risk of cerebrovascular, cardiovascular, and renal events.

Furthermore, the present invention includes a method for evaluating a long-term high onset risk of cerebrovascular, cardiovascular, and renal events by any of these methods, and for selecting an antithrombotic drug.

### ADVANTAGEOUS EFFECTS OF INVENTION

Using sCLEC-2 concentration alone, or in combination with D-Dimer concentration and/or platelet count, enables a convenient and accurate evaluation of the long-term onset risk of cerebrovascular, cardiovascular, and renal events. Here, the combination of sCLEC-2 concentration with D-Dimer concentration and/or platelet count refers to the combination of either D-Dimer concentration or platelet count with sCLEC-2 concentration, as well as the combination of D-Dimer concentration and platelet count with sCLEC-2 concentration. More specifically, this refers to the combination of blood D-Dimer concentration and blood sCLEC-2 concentration, the combination of platelet count and blood sCLEC-2 concentration, and the combination of blood D-Dimer concentration, platelet count, and blood sCLEC-2 concentration.

### BRIEF DESCRIPTION OF THE DRAWINGS

[Fig. 1] Figure 1 is a graph showing the incidences of cerebrovascular, cardiovascular, and renal events based on the tertiles of blood sCLEC-2 concentrations in 453 CKD patients.
[Fig. 2] Figure 2 is a graph showing the Kaplan-Meier curves based on the tertiles of blood sCLEC-2 concentrations in 453 CKD patients.
[Fig. 3] Figure 3 is a graph showing the incidences of cerebrovascular, cardiovascular, and renal events based on the combination of blood sCLEC-2 concentration with blood D-Dimer concentration in 453 CKD patients.
[Fig. 4] Figure 4 is a graph showing the Kaplan-Meier curves based on the combination of blood sCLEC-2 concentration with blood D-Dimer concentration in 453 CKD patients.
[Fig. 5] Figure 5 is a graph showing the incidences of cerebrovascular, cardiovascular, and renal events based on the tertiles of (blood sCLEC-2 concentration) × (blood D-Dimer concentration) in 453 CKD patients.
[Fig. 6] Figure 6 is a graph showing the Kaplan-Meier curves based on the tertiles of (blood sCLEC-2 concentration) × (blood D-Dimer concentration) in 453 CKD patients.
[Fig. 7] Figure 7 is a graph showing the incidences of cerebrovascular, cardiovascular, and renal events based on the tertiles of (blood D-Dimer concentration) × (blood sCLEC-2 concentration) / (platelet count) in 453 CKD patients.
[Fig. 8] Figure 8 is a graph showing the Kaplan-Meier curves based on the tertiles of (blood D-Dimer concentration) × (blood sCLEC-2 concentration) / (platelet count) in 453 CKD patients.

### DESCRIPTION OF EMBODIMENTS

Hereinafter, embodiments of the method of the present invention for evaluating the long-term high onset risk of cerebrovascular, cardiovascular, and renal events will be described in detail, using the measurement and analysis of blood sCLEC-2 and other markers of chronic kidney disease (CKD) patients as examples, but the applications of the present invention are not limited to the examples.

The method of the first embodiment of the present invention comprises the step of measuring the concentration of sCLEC-2 in a blood sample collected from a subject to be evaluated for the risk of cerebrovascular, cardiovascular, and renal events (hereinafter referred to as the subject). When evaluating the long-term onset risk of cerebrovascular, cardiovascular, and renal events based solely on sCLEC-2 concentration, an appropriate cutoff value can be set, for example, at 200 pg/mL or higher, and subjects exhibiting values equal to or higher than the cutoff value can be evaluated as having a higher long-term onset risk of cerebrovascular, cardiovascular, and renal events. Subjects exhibiting values lower than the cutoff value can be evaluated as having a lower long-term onset risk of cerebrovascular, cardiovascular, and renal events.

The method of the second embodiment of the present invention comprises the step of measuring the concentrations of sCLEC-2 and D-Dimer in a blood sample collected from the subject. When the long-term onset risk of cerebrovascular, cardiovascular, and renal events is evaluated based on the combination of sCLEC-2 concentration with D-Dimer concentration, for example, an appropriate cutoff value can be set for each, and if either exceeds the cutoff value, or if both exceed the cutoff values, the subject can be evaluated as having a higher long-term onset risk of cerebrovascular, cardiovascular, and renal events. The cutoff value for sCLEC-2 concentration in this case may be the value used when evaluating based solely on sCLEC-2 concentration. The cutoff value for D-Dimer concentration may be preferably 1.0 µg/mL or more, which is the upper limit for healthy individuals, and more preferably 1.45 µg/mL or more.

As another evaluation method of the second embodiment, when evaluating based on the value calculated by multiplying sCLEC-2 concentration by D-Dimer concentration [i.e., (sCLEC-2 concentration) × (D-Dimer concentration)], an appropriate cutoff value for the index value can be set, subjects exhibiting values equal to or higher than the cutoff value can be evaluated as having a higher long-term onset risk of cerebrovascular, cardiovascular, and renal events, and subjects exhibiting values lower than the cutoff value can be evaluated as having a lower long-term onset risk of cerebrovascular, cardiovascular, and renal events.

The method of the third embodiment of the present invention comprises the step or measuring the concentration of sCLEC-2 and platelet count in a blood sample collected from the subject. Furthermore, in the method of the third embodiment of the present invention, in addition to the sCLEC-2 concentration and platelet count, the concentration of D-Dimer in the blood sample from the subject can also be measured.

In the method of the third embodiment of the present invention, when the long-term onset risk of cerebrovascular, cardiovascular, and renal events is evaluated based on the combination of sCLEC-2 concentration with platelet count, the evaluation can be performed, for example, by using the evaluation method (for example, based solely on sCLEC-2 concentration) and the cutoff value in the method of the first embodiment and further using platelet count; or by using an evaluation method by combining sCLEC-2 concentration with platelet count and using a cutoff value(s). As a specific calculation formula for the evaluation method by combining sCLEC-2 concentration with platelet count, for example, an index value of "(sCLEC-2 concentration) / (platelet count)" can be used.

In another method of the third embodiment of the present invention, when the long-term onset risk of cerebrovascular, cardiovascular, and renal events is evaluated based on the combination of sCLEC-2 concentration, D-Dimer concentration, and platelet count, the evaluation can be performed, for example, by using the evaluation method (for example, based on the combination of sCLEC-2 concentration with D-Dimer concentration) and the cutoff values in the method of the second embodiment and further using platelet count; or by adding D-Dimer concentration to the evaluation method and the cutoff value(s) by combining sCLEC-2 concentration with platelet count. As a specific calculation formula for the evaluation method by combining sCLEC-2 concentration, D-Dimer concentration, and platelet count, for example, an index value of "(D-Dimer concentration) × (sCLEC-2 concentration) / (platelet count)" can be used.

Since sCLEC-2 is generated from platelets, sCLEC-2 concentration shows a weak correlation with platelet count. Therefore, it has been reported that the index value "(sCLEC-2 concentration) / (platelet count)", which represents the sCLEC-2 value per platelet, better indicates the degree of platelet activation than sCLEC-2 concentration alone (Patent Literature 4), and this can be referred to as appropriate.

For example, an appropriate cutoff value for the value "(sCLEC-2 concentration) / (platelet count)" calculated by dividing sCLEC-2 concentration by platelet count can be set, subjects exhibiting values higher than the cutoff value can be evaluated as having a higher long-term onset risk of cerebrovascular, cardiovascular, and renal events.

Furthermore, an appropriate cutoff value can be set for each of the "(sCLEC-2 concentration) / (platelet count)" value and D-Dimer concentration, and if either exceeds the cutoff value, or if both exceed the cutoff values, the subject can be evaluated as having a higher long-term onset risk of cerebrovascular, cardiovascular, and renal events. The cutoff value for "(sCLEC-2 concentration) / (platelet count)" in this case can be appropriately set and used based on sCLEC-2 concentration and platelet count. The cutoff value for D-Dimer concentration may be preferably 1.0 µg/mL or more, which is the upper limit for healthy individuals, and more preferably 1.45 µg/mL or more.

Although, in the specific example described above, the method of using platelet count was explained using the value calculated by dividing sCLEC-2 concentration by platelet count, for the value represented by the calculation formula explained herein, for example, the following Formula (1): $\left\{\left(soluble CLEC-2\right)\times \left(D-Dimer concentration\right)\right\} / \left(platelet count\right)$ the order of operations within Formula (1) is arbitrary, so long as the rules of calculation well known to those skilled in the art (rules of order of operations, or rules for the four arithmetic operations) are followed. That is, so long as the calculation results are the same, the order of the items used in the calculation is unrestricted, and the combinations of values used to set the cutoff values are also unrestricted.

For example, when using the items described in Formula (1), the long-term high risk of cerebrovascular, cardiovascular, and renal events can be evaluated by:
(1) the value calculated by multiplying "the value calculated by dividing soluble CLEC-2 concentration in the blood of a subject by platelet count" by D-Dimer concentration;
(2) the value calculated by multiplying "the value calculated by dividing D-Dimer concentration in the blood of a subject by platelet count" by soluble CLEC-2 concentration; or
(3) the value calculated by dividing "the value calculated by multiplying soluble CLEC-2 concentration in the blood of a subject by D-Dimer concentration in the blood of a subject" [or (3') "the value calculated by multiplying D-Dimer concentration in the blood of a subject by soluble CLEC-2 concentration in the blood of a subject"] by platelet count.

As another evaluation method of the third embodiment, when evaluating based on the value calculated by dividing the value calculated by multiplying sCLEC-2 concentration by D-Dimer concentration by platelet count [i.e., (D-Dimer concentration) × (sCLEC-2 concentration) / (platelet count)], an appropriate cutoff value for the value can be set, subjects exhibiting values equal to or higher than the cutoff value can be evaluated as having a higher long-term onset risk of cerebrovascular, cardiovascular, and renal events, and subjects exhibiting values lower than the cutoff value can be evaluated as having a lower long-term onset risk of cerebrovascular, cardiovascular, and renal events.

The term "cerebrovascular, cardiovascular, and renal events" as used herein refers to the occurrence of symptoms or therapeutic interventions in cerebrovascular and cardiovascular diseases. Examples of the "cerebrovascular, cardiovascular, and renal events" include cerebrovascular and cardiovascular death (fatal myocardial infarction, fatal stroke, sudden cardiac death), non-fatal myocardial infarction, unstable angina, ischemic stroke, transient ischemic attack, non-fatal stroke, percutaneous coronary intervention (PCI), coronary artery bypass grafting (CABG; also referred to as aortocoronary (AC) bypass grafting), other cardiovascular reconstructive procedures, initiation of hemodialysis, arteriosclerosis obliterans, critical limb ischemia, and aortic dissection.

In the present specification, specimens collected from subjects are preferably, for example, blood samples, anticoagulant-treated plasma or whole blood, or serum, which are collected from humans, but may be collected from other mammals such as rats, mice, monkeys, and dogs that are susceptible to developing cerebrovascular, cardiovascular, and renal events. Subjects are preferably patients suspected of having, or diagnosed with, diseases considered to have a high risk of developing particularly cerebrovascular, cardiovascular, and renal events. Risk factors for cerebrovascular, cardiovascular, and renal events include, for example, patients with chronic kidney disease, diabetes, dyslipidemia, hypertension, chronic maintenance dialysis, atherosclerotic diseases, or the like, obesity, and smokers, and patients with a history of cerebrovascular, cardiovascular, and renal events are preferred.

As the method for collecting specimens from subjects, known collection methods may be used as appropriate depending on the measurement items. For example, when measuring sCLEC-2 concentration, specimens can be collected using blood collection tubes for plasma collection. Citrate-containing blood collection tubes with low residual platelets are preferable, but heparin or EDTA-containing tubes are also acceptable. when measuring D-Dimer concentration, citrate or EDTA-containing blood collection tubes are preferable. When measuring platelet count, EDTA-containing blood collection tubes are preferable. The same blood collection tubes may be used, or separate tubes may be used for each.

The method for measuring sCLEC-2 concentration is not particularly limited, but immunological methods using antibodies that recognize sCLEC-2 are preferred. As the methods for immunologically detecting protein, any method may be used, so long as it is a known and commonly used method. Such methods include, for example, immunoassays using labeled antibodies, such as enzyme-linked immunosorbent assay, chemiluminescent immunoassay, electrochemiluminescent immunoassay, fluorescent immunoassay, radioimmunoassay, immunochromatography, and Western blotting; or latex agglutination method, and immunoturbidimetric assay. However, a measurement method with sufficient sensitivity to measure sCLEC-2 concentration in patients or healthy individuals should be used, and therefore, a highly sensitive measurement method is desirable, and chemiluminescent immunoassay, electrochemiluminescent immunoassay, fluorescent immunoassay, and the like are most preferably used.

The method for measuring D-Dimer concentration is not particularly limited, but may be a known immunological measurement method, such as enzyme-linked immunosorbent assay, chemiluminescent enzyme immunoassay, chemiluminescent immunoassay, fluorescent immunoassay, bioluminescent immunoassay, electrochemiluminescent immunoassay, latex agglutination method, and immunochromatography.

The method for measuring platelet count is not particularly limited, but conventional and known methods may be used as appropriate.

The term "CLEC-2" as used herein refers to a platelet-activating receptor belonging to the C-type lectin family, which is normally present on the platelet membrane, but is released into the blood upon platelet activation.

The term "soluble CLEC-2 (sCLEC-2)" as used herein refers to CLEC-2 or CLEC-2-derived molecules that are released from platelets and detected in the blood.

sCLEC-2 is reported to contain proteins with molecular weights of approximately 40 kDa, 32 kDa, and 25 kDa, as determined by SDS-polyacrylamide gel electrophoresis (SDS-PAGE) under reducing conditions. The proteins with molecular weights of approximately 40 kDa and 32 kDa are present on the platelet membrane surface, and are presumed to be released in a state incorporated into microparticles produced upon platelet activation. It is thought that sugar chains are added to these proteins. On the other hand, the protein with a molecular weight of approximately 25 kDa is thought to be cleaved by proteases upon platelet activation and released from the platelets. In the present invention, the amount of sCLEC-2 as described above is measured. sCLEC-2 may be detected as a mixture of the protein with a molecular weight of approximately 40 kDa, the protein with a molecular weight of approximately 32 kDa, and the protein with a molecular weight of approximately 25 kDa, or it may be detected solely as the protein with a molecular weight of approximately 25 kDa.

The concentration of sCLEC-2 measured from the subject's specimen is typically expressed in pg/mL, but any unit such as ng/L or ng/mL is acceptable. The concentration of D-Dimer is typically expressed in µg/mL or ng/mL, but any unit such as ng/L is acceptable. The D-Dimer concentration may be expressed in weight unit or fibrinogen equivalent unit (FEU) of D-Dimer. The platelet count is expressed in, for example, 1,000 cells/µL or 10,000 cells/µL, but any unit is acceptable. The index values such as (sCLEC-2 concentration) × (D-Dimer concentration) or (D-Dimer concentration) × (sCLEC-2 concentration) / (platelet count) can take various numerical values depending on the units used for each marker. However, this is essentially the same regardless of the combination of units used and can be set appropriately by referring to known methods. Additionally, similar index values combining sCLEC-2 concentration, D-Dimer concentration, or platelet count can also be utilized.

For setting the cutoff value, measurement data from the group that experienced cerebrovascular, cardiovascular, and renal events after a certain period in multiple populations are compared with those that did not experience such events, and the cutoff value is set appropriately using statistical methods. The cutoff value can be set appropriately by referring to known methods. For example, from measured sCLEC-2 concentration or D-Dimer concentration values, or index values such as For example, from measured sCLEC-2 concentration or D-Dimer concentration values, or index values such as (sCLEC-2 concentration) × (D-Dimer concentration) or (D-Dimer concentration) × (sCLEC-2 concentration) / (platelet count), appropriate stratification is performed using, for example, a two-quantile method, a tertile method, or a quartile method. A particularly high-risk group can be identified, and the cutoff value can be determined based on its threshold. As a threshold, it is acceptable to determine the threshold by setting a high-risk group without being fixated on dividing into equal parts. Alternatively, an analysis can be performed to create a ROC curve (Receiver Operating Characteristic Curve), allowing the cutoff value to be determined based on the sensitivity and specificity of the risk prediction.

In the case of sCLEC-2 concentration alone, (sCLEC-2 concentration) × (D-Dimer concentration), or (D-Dimer concentration) × (sCLEC-2 concentration) / (platelet count), subjects exhibiting values higher than the cutoff value can be judged as having a higher risk, and subjects exhibiting values lower than the cutoff value can be judged as having a lower risk.

In the case of the combination of sCLEC-2 concentration and D-Dimer concentration, the cutoff value is set for each. If both of sCLEC-2 concentration and D-Dimer concentration are higher than each cutoff value, the subject can be judged as having a higher risk, and if both are lower than each cutoff value, the subject can be judged as having a lower risk. Alternatively, if either is higher than each cutoff value, the subject can be judged as having a higher risk, and if either is lower than each cutoff value, the subject can be judged as having a lower risk.

Additionally, when stratification is performed into three or more groups, such as high risk, medium risk, and low risk, it is also possible to set two or more cutoff values.

The calculation of the aforementioned indicator values is presumed to be often performed using measurements obtained from clinical testing equipment that measures sCLEC2 concentration or D-Dimer concentration, and measurements obtained from a hematology analyzer that measure platelet count. This calculation is preferably performed, for routine clinical practice, automatically within hospital systems, such as a laboratory system, hospital system, or electronic medical record system, which connects to two or more measuring devices. Alternatively, a system which links data from two or more measuring devices may be constructed, or it is possible to construct equipment capable of simultaneously measuring sCLEC2 concentration and D-Dimer concentration, or equipment capable of simultaneously measuring sCLEC2 concentration, D-Dimer concentration, and platelet count. Additionally, manual calculations can also be performed using two or more data points.

The method of the present invention for evaluating the long-term onset risk of cerebrovascular, cardiovascular, and renal events is used as an aid for physicians in making diagnoses.

The term "long-term onset risk of cerebrovascular, cardiovascular, and renal events" as used herein refers to the possibility of developing cerebrovascular, cardiovascular, and renal events several months or more from the time of specimen collection, for example, six months or more from the time of specimen collection. For a longer-time evaluation, it refers to the possibility of developing cerebrovascular, cardiovascular, and renal events up to 12 months, further up to 18 months, and still further up to 24 months.

When a subject is evaluated by the present invention as having the long-term high onset risk of cerebrovascular, cardiovascular, and renal events, it is suspected that the subject has a tendency toward thrombosis, and antithrombotic therapy can be recommended for the subject. For selecting antithrombotic therapy, antiplatelet agents (for example, aspirin or clopidogrel) or anticoagulants (for example, warfarin or DOACs) can be selected based on sCLEC-2, D-Dimer, and/or platelet measurements, or index values, along with the patient's background information. Since antithrombotic therapy carries side effects such as bleeding, it is highly desirable to be able to select patients for whom antithrombotic therapy can be recommended, thereby enhancing the benefits for these patients in prevention.

The present invention includes, for example, a system capable of implementing the method of the present invention described above; an electronic medical record, clinical laboratory equipment, or an in-hospital laboratory system equipped with the system; and a kit capable of implementing the method of the present invention.

The system of the present invention includes, for example, a system for measuring (or determining) the aforementioned index values; a system for evaluating the long-term high onset risk of cerebrovascular, cardiovascular, and renal events; a system for assisting in an evaluation of the long-term high onset risk of cerebrovascular, cardiovascular, and renal events; a system for evaluating (or detecting) the long-term high onset risk of cerebrovascular, cardiovascular, and renal events; a system for measuring (or determining) the aforementioned index values for evaluating the long-term high onset risk of cerebrovascular, cardiovascular, and renal events. The specific index values may be the combinations of soluble CLEC-2 concentration with D-Dimer concentration and/or platelet count, and, more specifically, for example, (sCLEC-2 concentration) × (D-Dimer concentration), (sCLEC-2 concentration) / (platelet count), or (D-Dimer concentration) × (sCLEC-2 concentration) / (platelet count).

Any system of the present invention may comprise:
(1) a storage means capable of storing a soluble CLEC-2 concentration, and a D-Dimer concentration and/or a platelet count in a blood sample derived from a subject;
(2) a calculation means capable of calculating an index value combining the soluble CLEC-2 concentration with the D-Dimer concentration and/or the platelet count;
(3) a comparison means capable of comparing the index value obtained in (2) with a cutoff value (for example, an index value of the comparison group); and
(4) a display means capable of displaying a result obtained from the comparison.

Alternatively, any system of the present invention may be equipped with a computer comprising a processor and a memory controlled by the processor, and the memory may store a program that causes the computer to execute the following steps (1) to (4):
(1) a storage step of storing in the memory a soluble CLEC-2 concentration, and a D-Dimer concentration and/or a platelet count in a blood sample derived from a subject;
(2) a calculation step of calculating an index value combining the soluble CLEC-2 concentration with the D-Dimer concentration and/or the platelet count;
(3) a comparison step of comparing the index value obtained in the calculation step with a cutoff value (for example, an index value of the comparison group); and
(4) a display step of displaying a result obtained from the comparison step.

The electronic medical record, clinical laboratory equipment, an in-hospital laboratory system, or the like of the present invention may be equipped with the system of the present invention.

The method of the present invention may be implemented using the system of the present invention, or the electronic medical record, clinical laboratory equipment, an in-hospital laboratory system, or the like of the present invention (hereinafter collectively referred to as the system or the like of the present invention). The index values that can be measured (or determined) using the system or the like of the present invention; the "conditions, diseases, and the like" that can be evaluated using the system or the like of the present invention, and their "criteria of evaluation, judgment, decision, and the like"; the measurement methods for "soluble CLEC-2 concentration, D-Dimer concentration, and/or platelet count"; and the like can be understood by applying the explanations described above regarding the method of the present invention.

The kit of the present invention may include, for example, a soluble CLEC-2 measurement reagent, a D-Dimer measurement reagent, a platelet count measurement reagent, and includes at least a soluble CLEC-2 measurement reagent. Furthermore, the kit of the present invention may include an instruction manual that describes a relationship of a long-term high onset risk of cerebrovascular, cardiovascular, and renal events, with soluble CLEC-2 concentration, D-Dimer concentration, or platelet count, or index values combining soluble CLEC-2 concentration with D-Dimer concentration and/or platelet count. The instruction manual may also include the calculation methods for the index values.

Additionally, the instruction manual included in the kit of the present invention is not particularly limited, so long as it refers, at least, to the relationship of the long-term high onset risk of cerebrovascular, cardiovascular, and renal events, with soluble CLEC-2 concentration, or index values combining soluble CLEC-2 concentration with D-Dimer concentration and/or platelet count. In addition to the reference to the aforementioned relationship, it may include, for example, explanations of the procedures for immunological assays using the kit of the present invention; explanations of the procedures for evaluating the long-term high onset risk of cerebrovascular, cardiovascular, and renal events based on the obtained measurements; precautions regarding storage, handling, or the like of the kit itself; and the like.

### EXAMPLES

The present invention will now be further illustrated by, but is by no means limited to, the following Examples.

### <<Example 1: Measurement of sCLEC-2, D-Dimer, and platelet count and other data collection in patients with chronic kidney disease>>

### Example 1-1: Measurement of sCLEC-2

### (Preparation of CLEC-2 measurement reagents)

· Sample dilution solution: A sample dilution solution was prepared by combining 0.1 mol/L HEPES buffer (pH 7.5) containing preservatives with sodium octanoate and n-octyl-β-D-glucoside (OG) at final concentrations of 2% and 0.5%, respectively.

The antibodies contained in the following reagents were prepared as follows, using antibodies described in the Examples of Japanese Patent No. 6078845.
· First antibody solution: Magnetic latex particles (JSR Corporation) were conjugated with a mouse monoclonal antibody (11D5) recognizing sCLEC-2, and dispersed in 0.01 mol/L MES buffer (pH 6.0) containing preservatives.
· Second antibody solution: Another mouse monoclonal antibody (11E6) recognizing sCLEC-2 was labeled with alkaline phosphatase (ALP) using a maleimide method, and dispersed in 0.01 mol/L MES buffer (pH 6.5) containing preservatives.
· Luminescent substrate solution: Disodium 2-chloro-5-(4-methoxyspiro{1,2-dioxetane-3,2'-(5'-chloro)-tricyclo[3.3.1.13,7]decan}-4-yl)-1-phenylphosphate (CDP-Star (registered trademark): Applied Biosystems) was used.
· B/F washing solution: A buffer containing 0.1 mol/L citric acid (pH 6.5), 0.15 mol/L NaCl, and 0.1% Triton X-100 was used.

### (Measurement using measurement reagents)

Measurement of sCLEC-2 was performed using a fully automatic clinical laboratory system STACIA (registered trademark, manufactured by LSI Medience Corporation).

STACIA-specific bottles were filled with the prepared sample dilution solution, first antibody solution (magnetic latex reagent), and second antibody solution (enzyme-labeled antibody reagent), respectively, and set into the device. The following measurements were performed according to the operating method of the device.

Specifically, 10 µL of each sample collected as citrate plasma was mixed with 40 µL of the sample dilution solution. After heating at 37°C for several minutes, 25 µL of the first antibody solution (magnetic latex reagent) was added and the mixture was heated at 37°C for several minutes. Next, B/F separation was performed. Then, 50 µL of the second antibody solution (enzyme-labeled antibody reagent) was added and heated at 37°C for several minutes. After performing B/F separation again, 100 µL of the luminescent substrate solution was added. After reacting at 37°C for several minutes, the signal intensity (counts) was measured.

### Example 1-2: Measurement of D-Dimer

Measurement of D-Dimer was performed using an LPIA-Genesis D-Dimer reagent (manufactured by LSI Medience Corporation) on the STACIA.

### Example 1-3: Measurement of platelet count

Platelet counts were measured using an automated hematology analyzer (Sysmex Corporation).

### Example 1-4: Measurement of other items

B-type natriuretic peptide (BNP) was measured using a reagent manufactured by LSI Medience Corporation. Hemoglobin, blood glucose level, and creatinine (for eGFR calculation) were measured using reagents conforming to standard methods.

### Example 1-5: Specimens and study protocols

Subjects to be measured were 453 patients with chronic kidney disease (CKD) undergoing outpatient treatment (age: 64 ± 15 years, estimated glomerular filtration rate (eGFR): 49 ± 25 mL/min/1.73 m²). Patients with CKD stage 5 (eGFR: lower than 15 mL/min/1.73 m²) and those aged 85 years or older were excluded. Blood samples were collected for measurement during the outpatient visit, and sCLEC-2, D-Dimer, platelet count, B-type natriuretic peptide (BNP), hemoglobin, blood glucose level, and creatinine (for eGFR calculation) were measured. Furthermore, past medical history was investigated for myocardial infarction, stroke, heart failure, hypertension, diabetes, and dyslipidemia. The endpoint was defined as cerebrovascular, cardiovascular, and renal events (new onset of cerebrovascular or cardiovascular disease, or initiation of hemodialysis) within the 24-month observation period.

### <<Example 2: Evaluation of long-term onset risk of cerebrovascular, cardiovascular, and renal events in CKD patients>>

Results from the analysis conducted according to Example 1 showed that 52 cases (11.5%) of cerebrovascular, cardiovascular, and renal events occurred during the 24-month observation period. The event group showed significantly higher levels of sCLEC-2, D-Dimer, BNP, and blood glucose level compared to the non-event group. On the other hand, eGFR and hemoglobin levels were significantly lower in the event group. Results of Cox proportional hazards multivariate analysis revealed that sCLEC-2, D-Dimer, and blood glucose levels, and history of stroke were independent predictors of cerebrovascular, cardiovascular, and renal events.

### <<Example 3: Evaluation of long-term high onset risk of cerebrovascular, cardiovascular, and renal events in CKD patients based solely on sCLEC-2 concentration>>

When CKD patients were divided into three groups using a sCLEC-2 value tertile method, the event incidences for each tertile were as shown in Table 1.

**[Table 1]**

| | | | | |
|---|---|---|---|---|
| Incidences of cerebrovascular, cardiovascular, and renal events (%) | sCLEC-2 (pg/mL) | | | |
| | T1 <250 (n=148) | T2 250-385 (n=152) | T3 >385 (n=153) | P value |
| | 4.7 | 14.5 | 15.0 | 0.005 |

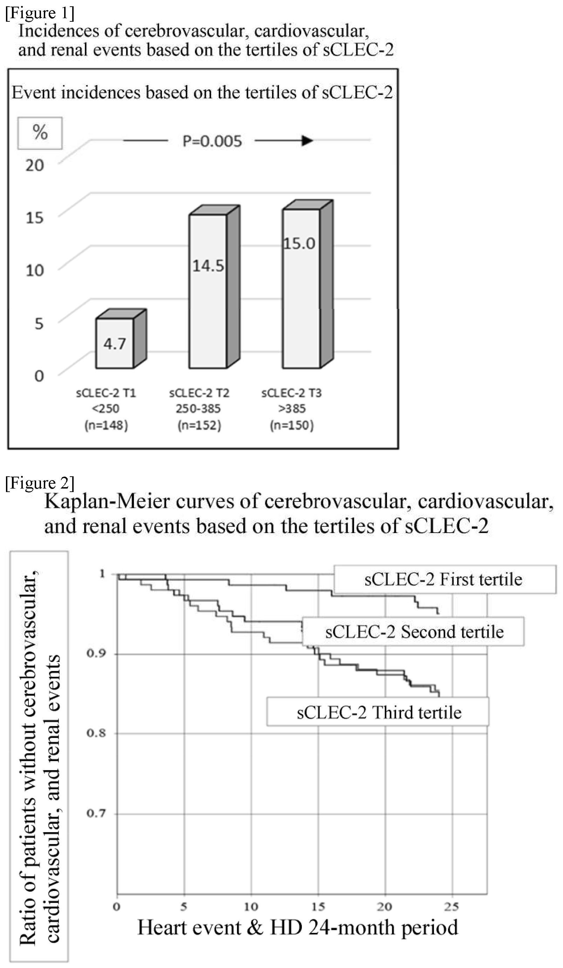

This is shown in Figure 1 as a graph. For sCLEC-2 values of 250 pg/mL or higher, the long-term risk over 24 months was shown to be approximately three times higher compared to values below 250 pg/mL.

Figure 2 shows the Kaplan-Meier curves for this CDK case cohort. According to the Kaplan-Meier curves, in cases with sCLEC-2 values of 250 pg/mL or higher, the incidence of cerebrovascular, cardiovascular, and renal events increased almost linearly over 24 months (i.e., the proportion of subjects without events decreased linearly). This demonstrates that the sCLEC-2 value can be used to assess a long-term high onset risk of cerebrovascular, cardiovascular, and renal events.

### <<Example 4: Evaluation of long-term high onset risk of cerebrovascular, cardiovascular, and renal events in CKD patients based on the combination of sCLEC-2 concentration and D-Dimer concentration>>

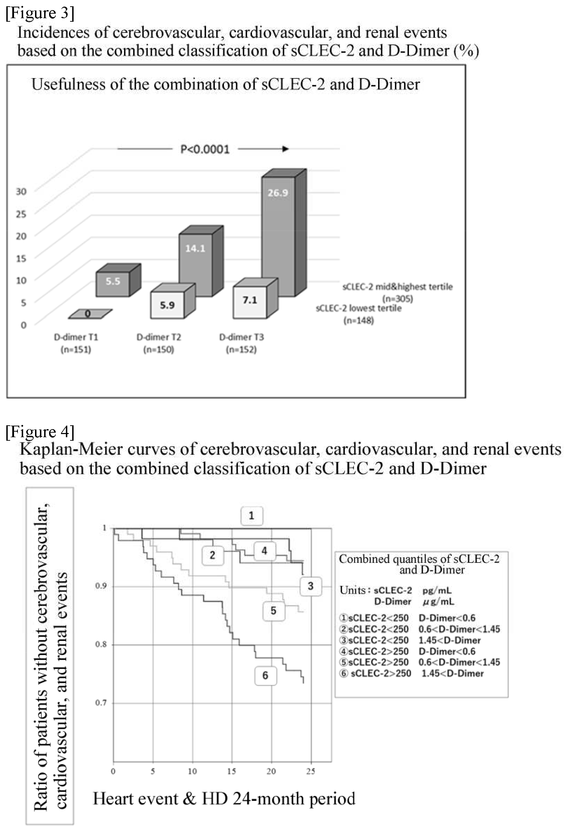

The CKD patient group was divided into two groups based on sCLEC-2 values: <250 pg/mL and ≥250 pg/mL, and further divided using the tertiles of D-Dimer (<0.6 µg/mL, 0.6-1.45 µg/mL, and ≥1.45 µg/mL). The incidences of cerebrovascular, cardiovascular, and renal events in each group are shown in Figure 3.

Furthermore, the Kaplan-Meier curves for this CDK case cohort are shown in Figure 4.

The group with sCLEC-2 ≥250 pg/mL and D-Dimer ≥1.45 µg/mL demonstrated an appropriately 27% probability of experiencing cerebrovascular, cardiovascular, and renal events within 24 months, indicating an extremely high long-term risk. Additionally, it is said that the upper limit for healthy individuals using this D-Dimer reagent is 1.0 µg/mL.

### <<Example 5: Evaluation of long-term high onset risk of cerebrovascular, cardiovascular, and renal events in CKD patients based on (sCLEC-2 concentration) × (D-Dimer concentration)>>

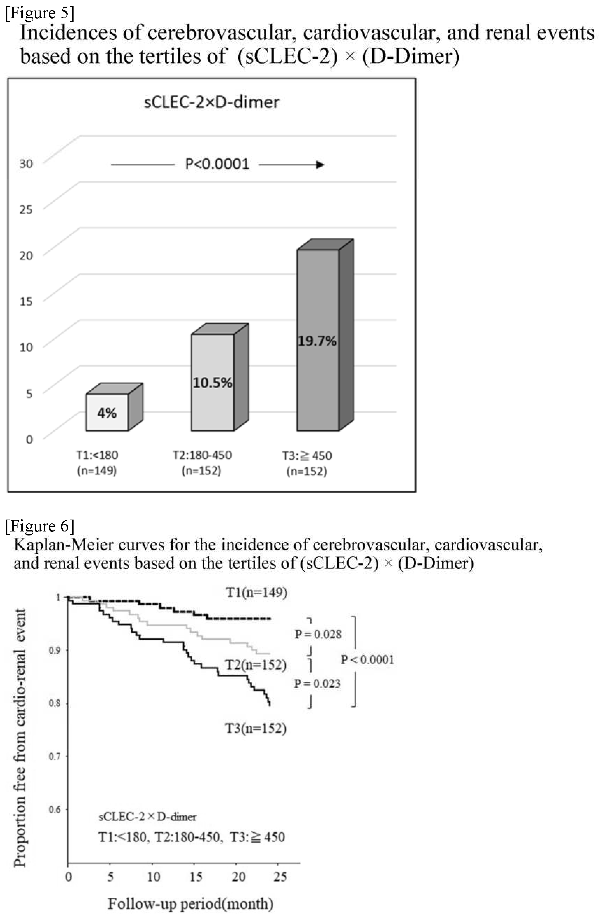

For each patient, we calculated the product of (sCLEC-2 concentration) and (D-Dimer concentration). Figure 5 shows the incidences of cerebrovascular, cardiovascular, and renal events based on the tertiles of this product value: <180, 180-450, and ≥450.

Furthermore, the Kaplan-Meier curves for this CDK case cohort are shown in Figure 6.

The group with a product of (sCLEC-2 concentration) and (D-Dimer concentration) of 450 or higher had a 19.7% probability of experiencing cerebrovascular, cardiovascular, and renal events within 24 months, indicating an extremely high long-term risk. Their risk was approximately five times higher than that of the group with a product of (sCLEC-2 concentration) and (D-Dimer concentration) of 180 or lower.

### <<Example 6: Evaluation of long-term high onset risk of cerebrovascular, cardiovascular, and renal events in CKD patients based on (D-Dimer concentration) × (sCLEC-2 concentration) / (platelet count)>>

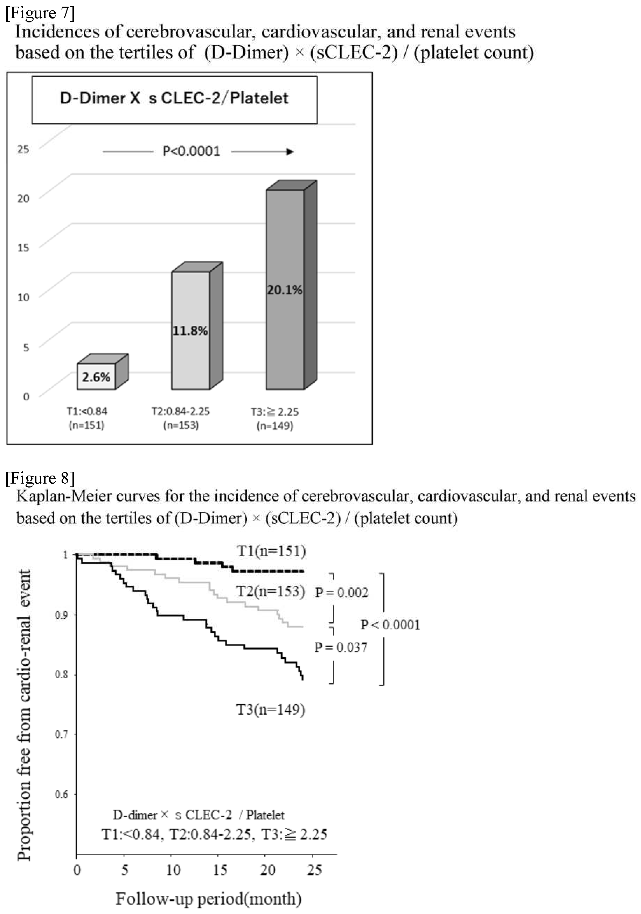

For each patient, we calculated the value of (D-Dimer concentration) × (sCLEC-2 concentration) / (platelet count). Figure 7 shows the incidences of cerebrovascular, cardiovascular, and renal events based on the tertiles of the value: <0.84, 0.84-2.25, and ≥2.25.

Furthermore, the Kaplan-Meier curves for these CDK patients are shown in Figure 8.

The group with a value of (D-Dimer concentration) × (sCLEC-2 concentration) / (platelet count) of 2.25 or higher had a 20.1% probability of experiencing cerebrovascular, cardiovascular, and renal events within 24 months, indicating an extremely high long-term risk. Their risk was approximately eight times higher than that of the group with a value of (D-Dimer concentration) × (sCLEC-2 concentration) / (platelet count) of 0.84 or lower.

### INDUSTRIAL APPLICABILITY

As described above, according to the present invention, the sCLEC-2 measurement value in the blood; the combination of the sCLEC-2 measurement value in the blood with the D-Dimer measurement value in the blood; the combination of the sCLEC-2 measurement value in the blood with platelet count; or the combination of the sCLEC-2 measurement value in the blood, the D-Dimer measurement value in the blood, and platelet count; serve as indicators for judging the long-term high onset risk of cerebrovascular, cardiovascular, and renal events. This enables the appropriate recommendation of antithrombotic therapy to enhance the benefits for patients in prevention. Measurement of sCLEC-2 in the blood is useful as a method for evaluating the long-term high onset risk of cerebrovascular, cardiovascular, and renal events.

## Claims

1. A method for evaluating a long-term high onset risk of cerebrovascular, cardiovascular, and renal events based on a soluble CLEC-2 concentration in a subject's blood.

2. The method according to claim 1, for evaluating a long-term high onset risk of cerebrovascular, cardiovascular, and renal events based on a soluble CLEC-2 concentration in a subject's blood, said method comprising:
(1) providing a blood sample derived from a subject;
(2) determining a soluble CLEC-2 concentration in the blood sample; and
(3) correlating the soluble CLEC-2 concentration with a long-term risk of onset for cerebrovascular, cardiovascular, and renal events in the subject.

3. The method according to claim 1 or 2, wherein the subject is a patient suspected of having a disease associated with a risk of cerebrovascular, cardiovascular, and renal events or a patient diagnosed with the disease.

4. The method according to any one of claims 1 to 3, wherein the disease associated with a risk of cerebrovascular, cardiovascular, and renal events is selected from the group consisting of chronic kidney disease, diabetes, hypertension, dyslipidemia, chronic maintenance dialysis, and atherosclerotic diseases.

5. The method according to any one of claims 1 to 4, wherein the long-term high onset risk of cerebrovascular, cardiovascular, and renal events is evaluated based on, in addition to the soluble CLEC-2 concentration in the subject's blood, a D-Dimer concentration in the subject's blood.

6. The method according to claim 5, wherein the long-term high onset risk of cerebrovascular, cardiovascular, and renal events is evaluated based on a value calculated by multiplying the soluble CLEC-2 concentration by the D-Dimer concentration in the subject's blood.

7. The method according to any one of claims 1 to 4, wherein the long-term high onset risk of cerebrovascular, cardiovascular, and renal events is evaluated based on, in addition to the soluble CLEC-2 concentration in the subject's blood, a subject's platelet count.

8. The method according to claim 7, wherein the long-term high onset risk of cerebrovascular, cardiovascular, and renal events is evaluated based on a value calculated by dividing the soluble CLEC-2 concentration in the subject's blood by the platelet count.

9. The method according to claim 7, wherein the long-term high onset risk of cerebrovascular, cardiovascular, and renal events is evaluated based on, in addition to the soluble CLEC-2 concentration in the subject's blood and the platelet count, a D-Dimer concentration in the subject's blood.

10. The method according to claim 9, wherein the long-term high onset risk of cerebrovascular, cardiovascular, and renal events is evaluated based on a value represented by the following formula (1): $\left\{\left(soluble CLEC-2\right)\times \left(D-Dimer concentration\right)\right\} / \left(platelet count\right)$

11. A method for evaluating a long-term high onset risk of cerebrovascular, cardiovascular, and renal events and selecting an antithrombotic drug, by the method according to any one of claims 1 to 10.

12. A system for evaluating a long-term high onset risk of cerebrovascular, cardiovascular, and renal events, said system comprising:
(1) a storage means capable of storing a soluble CLEC-2 concentration, and a D-Dimer concentration and/or a platelet count in a blood sample derived from a subject;
(2) a calculation means capable of calculating an index value combining the soluble CLEC-2 concentration with the D-Dimer concentration and/or the platelet count;
(3) a comparison means capable of comparing the index value obtained in (2) with a cutoff value; and
(4) a display means capable of displaying a result obtained from the comparison.

13. A system for evaluating a long-term high onset risk of cerebrovascular, cardiovascular, and renal events, equipped with a computer comprising a processor and a memory controlled by the processor, wherein the memory stores a program that causes the computer to execute the following steps (1) to (4):
(1) a storage step of storing in the memory a soluble CLEC-2 concentration, and a D-Dimer concentration and/or a platelet count in a blood sample derived from a subject;
(2) a calculation step of calculating an index value combining the soluble CLEC-2 concentration with the D-Dimer concentration and/or the platelet count;
(3) a comparison step of comparing the index value obtained in the calculation step with a cutoff value; and
(4) a display step of displaying a result obtained from the comparison step.

14. An electronic medical record, clinical laboratory equipment, or an in-hospital laboratory system equipped with the system according to claim 12 or 13.

15. A kit for evaluating a long-term high onset risk of cerebrovascular, cardiovascular, and renal events, said kit comprising:
(1) a measurement reagent for soluble CLEC-2; and
(2) an instruction manual that describes a relationship between a soluble CLEC-2 concentration or an index value, and a long-term high onset risk of cerebrovascular, cardiovascular, and renal events, said index value being a combination of the soluble CLEC-2 concentration with a D-Dimer concentration and/or a platelet count.
